# EUROPEAN PATENT APPLICATION

(11) **EP 4 481 030 A1**
(43) Date of publication of application: **25.12.2024**
(21) Application number: 23180390.9
(22) Date of filing: 20.06.2023
(51) Int. Cl.: C12M 1/26, B04B 5/04, C12M 1/00, C12N 1/02

(54) **METHOD OF OPERATING A BIOPROCESSING ARRANGEMENT COMPRISING A CLARIFICATION SET-UP TO REMOVE CELL DEBRIS FROM A CELL BROTH**

(71) Applicant: Sartorius Stedim Biotech GmbH, 37079 Göttingen (DE)
(72) Inventor: SABALLUS, Martin, 37079 Göttingen (DE); REGER, Lucas Nik, 37079 Göttingen (DE); KAMPMANN, Markus, 37079 Göttingen (DE); NIEMANN, Julia, 37079 Göttingen (DE)
(74) Representative: Gottschald Patentanwälte Partnerschaft mbB

(57) **Abstract**

The invention relates to a Method of operating a bioprocessing arrangement (1) comprising a clarification set-up (2) to remove cell debris from a cell broth (10), wherein the clarification set-up (2) comprises a fluidized bed centrifuge (8) with at least one centrifuge chamber (11), wherein the cell broth (10) is separated into at least a waste fraction (12) and an output fraction (13), wherein the separation of the cell broth (10) into the waste fraction (12) and the output fraction (13) comprises a loading step (14) during which the centrifuge chamber (11) is loaded with cells of the cell broth (10) up to a capacity below or equal to a maximum cell loading capacity of the centrifuge chamber (11), an overloading step (15) during which the centrifuge chamber (11) is further loaded with cell broth (10), thereby flushing out cells from the centrifuge chamber (11), collecting cells flushed out during the overloading step (15) as the waste fraction (12), and collecting cells from the chamber as the output fraction (13).

## Description

The present invention relates to a method of operating a bioprocessing arrangement comprising a clarification set-up to remove cell debris from a cell broth according to claim 1 and to a control system configured to control a bioprocessing arrangement to perform the proposed method according to claim 15.

The term "bioprocess" presently represents any kind of biotechnological process, in particular biopharmaceutical processes, using cells to produce a product or cultivating cells as the product. To achieve highly productive bioprocesses, maintaining cell viability is key. In existing cultivation scenarios elongation of proliferation and thus high cell viability over long time periods is usually achieved by media exchange in perfusion process scenarios using membrane devices for cell retention. However, these devices tend to block over time, lose unused media by back mixing, and are only capable to retain all (viable and dead) cells. Therefore, bleeding of cells (discharging a specific amount of cell broth) is needed from time to time to provide space for the dividing cells and to remove the already dead cells from the process. As a result, the process loses efficiency due to the unspecific removal of a part of the still productive cells.

It is a challenge to reduce the footprint of continuous bioprocesses and to increase the product output of non-continuous bioprocesses.

The invention is based on the problem of improving the known method such that a further optimization regarding the named challenge is reached.

The above-noted object is solved by the features of claim 1.

The main realization of the present invention is that a fluidized bed centrifuge is capable of increasing production efficiency in long-term cultivation scenarios by selective cell retention and thus bulk enrichment of viable cells while dead cells are discharged. This can be achieved by overloading the centrifuge chamber. Usually, a centrifuge chamber is loaded up to a maximum cell loading capacity as afterwards a significant increase in cells being flushed out of the chamber is observed. It has now been found that the cells being flushed out of the centrifuge chamber comprise significantly more dead cells than the cells remaining in the chamber. The reason for this effect is that dead cells are smaller and therefore accumulate at the elutriation boundary such that they are flushed out first if the chamber is overloaded. By using this effect on purpose, the overall viability of cells in a vessel like a bioreactor can be increased. This allows disposing of the bleeding in a continuous process or operating a batch process for a longer time. Compared to a filter the fluidized bed centrifuge as a multi-use apparatus has many advantages.

In detail, proposed is a method of operating a bioprocessing arrangement comprising a clarification set-up to remove cell debris from a cell broth, wherein the clarification set-up comprises a fluidized bed centrifuge with at least one centrifuge chamber, wherein the cell broth is separated into at least a waste fraction and an output fraction, wherein the separation of the cell broth into the waste fraction and the output fraction comprises a loading step during which the centrifuge chamber is loaded with cells of the cell broth up to a capacity below or equal to a maximum cell loading capacity of the centrifuge chamber, an overloading step during which the centrifuge chamber is further loaded with cell broth, thereby flushing out cells from the centrifuge chamber, collecting cells flushed out during the overloading step as the waste fraction, and collecting cells from the chamber as the output fraction.

The maximum cell loading capacity of the centrifuge chamber can be estimated experimentally with sufficient precision for the present purposes. The maximum cell loading capacity is reached when further loading of the centrifuge chamber with cell broth leads to a significant change in the increase of cells leaving the centrifuge chamber through its outlet. The paper "Martin Saballus, Lucy Nisser, Markus Kampmann, Gerhard Greller, A novel clarification approach for intensified monoclonal antibody processes with 100 million cells/mL using a single-use fluidized bed centrifuge, Biochemical Engineering Journal, Volume 167, 2021, 107887, ISSN 1369-703X, https:/Idoi.org/10.1016/j.bej.2020.107887" gives a formula for calculating the maximum cell broth capacity based on the maximum cell loading capacity and shows how reaching the maximum cell loading capacity greatly increases cells being flushed out of the centrifuge chamber.

According to claim 2, a valve may be switched as a transition between the loading step and the overloading step. The valve may be a valve rerouting the fluid output of the centrifuge chamber. Further, according to claim 3, during the loading step the fluid output of the chamber may be collected as a supernatant fraction separately from the waste fraction. It is preferred not to throw away the fluid output during the loading step. The proposed embodiment allows selectively declaring as waste the cells discharged during the overloading step which comprise significantly more dead cells than other fractions of the cell broth.

After the overloading step, the cells in the centrifuge chamber may be flushed out in a reverse direction (claim 4). In a very preferred embodiment according to claim 4, part of the supernatant fraction is used to flush out the cells, thereby not adding fresh culture medium to the cells.

Further taking the proposed method towards an increase of cell viability in a vessel, according to claim 5, the output fraction and optionally the supernatant fraction may be returned to the vessel. It is possible to return both fractions without diluting the cell broth and/or without washing the cells or combined with the addition of fresh culture medium. Claim 6 expands this embodiment towards further producing a product in the vessel after increasing the viability of the cells. According to claim 7, the increase in viability may be repeated several times. If a continuous or fed-batch process is desired, the volume removed with the waste fraction may be substituted with fresh culture medium.

Usually, a fluidized bed centrifuge is primed with buffer. An embodiment according to claim 9 includes priming the fluidized bed centrifuge with cell broth, which may be returned to the vessel afterwards, to reduce the media consumption and the footprint of the bioprocess.

The amount of cell broth input into the centrifuge chamber during the overloading step and therefore the amount of cells discharged from the centrifuge chamber may depend, according to claim 10, on a viability measurement of the fluid output of the centrifuge chamber.

Alternatively, according to claim 11, the amount of cells added to the centrifuge chamber during the overloading step may be predefined. The predefined amount may depend on a viability measurement of the cell broth. A good estimate for the number of cells added to the chamber after reaching the maximum cell loading capacity has been found to be around the number of dead cells in the centrifuge chamber. Then, the ratio of living cells to dead cells flushed out of the centrifuge chamber reaches a good compromise.

Repeatedly increasing the viability of the cells in the cell broth may be an automated process (claim 12).

In one embodiment according to claim 13, the fluidized bed centrifuge may be operating with the same parameters during the loading step and the overloading step to avoid destabilizing the fluidized bed, therefore performing the separation efficiently. In another embodiment according to claim 13, no washing of the cells in the centrifuge chamber is performed.

Claim 14 describes preferred embodiments of the bioprocess.

Another teaching according to claim 15, which is of equal importance, relates to a control system configured to control a bioprocessing arrangement to perform the proposed method.

All explanations given with regard to the proposed method are fully applicable.

In the following, embodiments of the invention are explained with respect to the drawing. The drawing shows in
- Fig. 1,: a bioprocessing arrangement arranged to repeatedly remove waste fractions from a bioreactor,
- Fig. 2,: loading, overloading and discharging of the centrifuge chamber and
- Fig. 3,: schematically the process of repeatedly removing waste fractions.

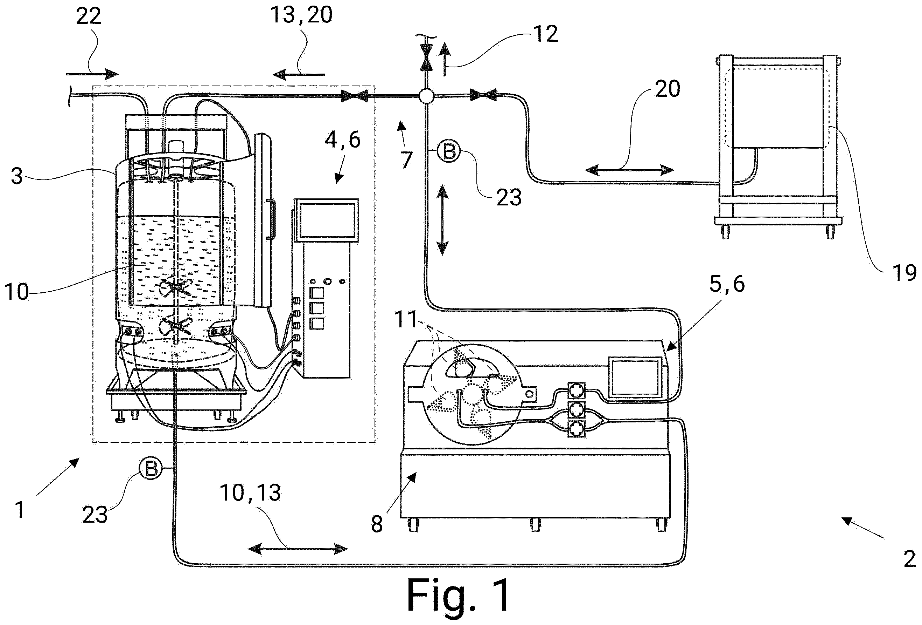

Fig. 1 shows a bioprocessing arrangement 1, here and preferably comprising a clarification set-up 2 and a bioreactor 3. Further optionally present components of the bioprocessing arrangement 1 include a bioreactor control unit 4 and a centrifuge control unit 5 as part of a control system 6. Also shown are a valve arrangement 7 and fluid lines connecting a fluidized bed centrifuge 8 of the clarification arrangement with the bioreactor 3.

The bioreactor 3 may be used to produce a product 9 like a monoclonal antibody by culturing cells in the bioreactor 3. Here and preferably, the production is a continuous process. Generally, the product 9 may be produced by the cells or the cells themselves.

Proposed is a method of operating a bioprocessing arrangement 1 comprising a clarification set-up 2 to remove cell debris, in particular dead cells, from a cell broth 10. During a continuous production, and any other production, dead cells and cell debris accumulates in the bioreactor 3, taking up space from the living cells and releasing inhibitory substances over the process duration, which reduces cell growth and production efficiency. For a continuous process it is necessary to remove inhibitory substances to keep the bioreactor 3 going. Therefore, it is particularly beneficial to remove cell debris, in particular dead cells, to reduce the release of those inhibitory substances. For a non-continuous process it can still be advantageous to remove cell debris to increase the efficiency of the process.

The clarification set-up 2 comprises a fluidized bed centrifuge 8 with at least one centrifuge chamber 11. As is generally known, fluidized bed centrifuges 8 work by creating a force equilibrium between centrifugal force and fluid flow to suspend cells inside the centrifuge chamber 11.

Here and preferably, cell broth 10 is contained in a vessel, here the bioreactor 3. It is now proposed that the cell broth 10 is separated into at least a waste fraction 12 and an output fraction 13.

The separation of the cell broth 10 into the waste fraction 12 and the output fraction 13 comprises a loading step 14 during which the centrifuge chamber 11 is loaded with cells of the cell broth 10 up to a capacity below or equal to a maximum cell loading capacity of the centrifuge chamber 11. Regarding the definition of the maximum cell loading capacity reference is made to the introductory part of the description. In one embodiment and/or if in doubt, the maximum cell loading capacity may be set as the capacity at which the output of the chamber reaches a total cell count per mL of less than 0.5 million cells per mL.

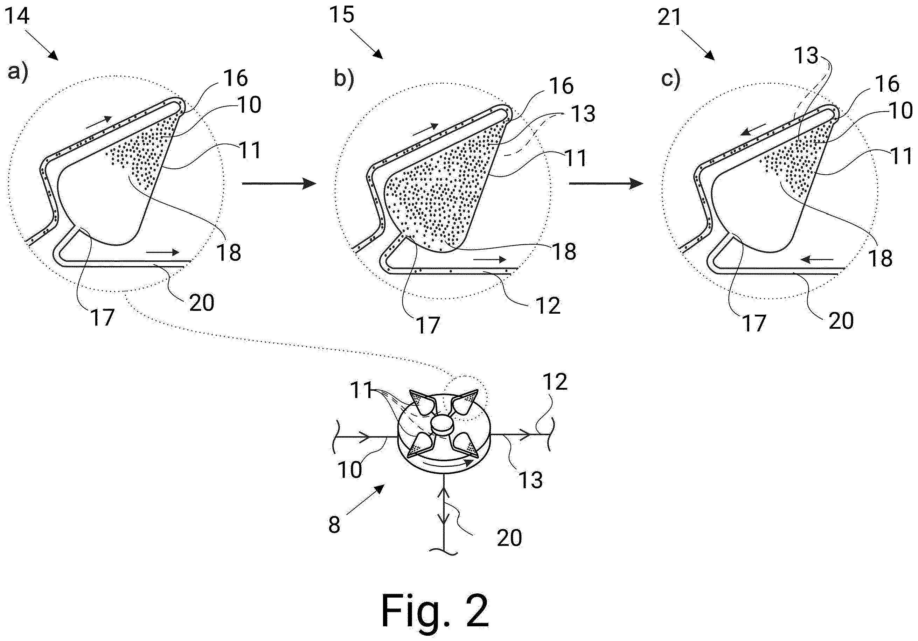

The separation of the cell broth 10 into the waste fraction 12 and the output fraction 13 further comprises an overloading step 15 during which the centrifuge chamber 11 is further loaded with cell broth 10, thereby flushing out cells from the centrifuge chamber 11. Fig. 2 shows the loading step 14 in which the centrifuge chamber 11 is loaded with cells from the cell broth 10 while fluid, then supernatant, from the cell broth 10 is passing through the centrifuge chamber 11. In detail, Fig. 2 shows a chamber inlet 16 through which the cell broth 10 enters the centrifuge chamber 11, a chamber outlet 17 through which parts of the cell broth 10 leave the centrifuge chamber 11 and different loading and operating states of the fluidized bed centrifuge 8. In Fig. 2a), the centrifuge chamber 11 is not fully loaded, and the elutriation boundary 18 is still well before the widest part of the centrifuge chamber 11. Fig. 2b) shows that the centrifuge chamber 11 has been further loaded, and the elutriation boundary 18 has roughly reached the widest part of the centrifuge chamber 11. Smaller cells are being flushed out of the centrifuge chamber 11 as they are displaced by other cells. As dead cells are smaller, a relatively higher amount of dead cells is flushed out. Fig. 2c) shows a consecutive reverse operation of the centrifuge chamber 11 flushing the remaining cells out of the centrifuge chamber 11 through the chamber inlet 16. Here and preferably, the loading step 14 and the overloading step 15 are distinct steps with a transition discernible on a physical level and/or a control level of the bioprocessing arrangement 1.

The separation of the cell broth 10 into the waste fraction 12 and the output fraction 13 further comprises collecting cells flushed out during the overloading step 15, preferably all cells flushed out during the overloading step 15, as the waste fraction 12. The waste fraction 12 may then be further processed on a downstream level or disposed with. The separation of the cell broth 10 into the waste fraction 12 and the output fraction 13 further comprises collecting cells, preferably all cells, from the chamber as the output fraction 13 (Fig. 2c)). The output fraction 13 and the waste fraction 12 are distinct, separate fractions. It should be understood that the amount of cells flushed out during the overloading step 15 is substantially greater than the usual amount of cells being flushed out during the loading step 14. Here and preferably, the waste fraction 12 and/or the liquid output of the centrifuge chamber 11 being added to the waste fraction 12 at some point comprises at least cells per volume equal to or above 10 % of the cells per volume of the cell broth 10, for example at least 5,000,000 cells per mL.

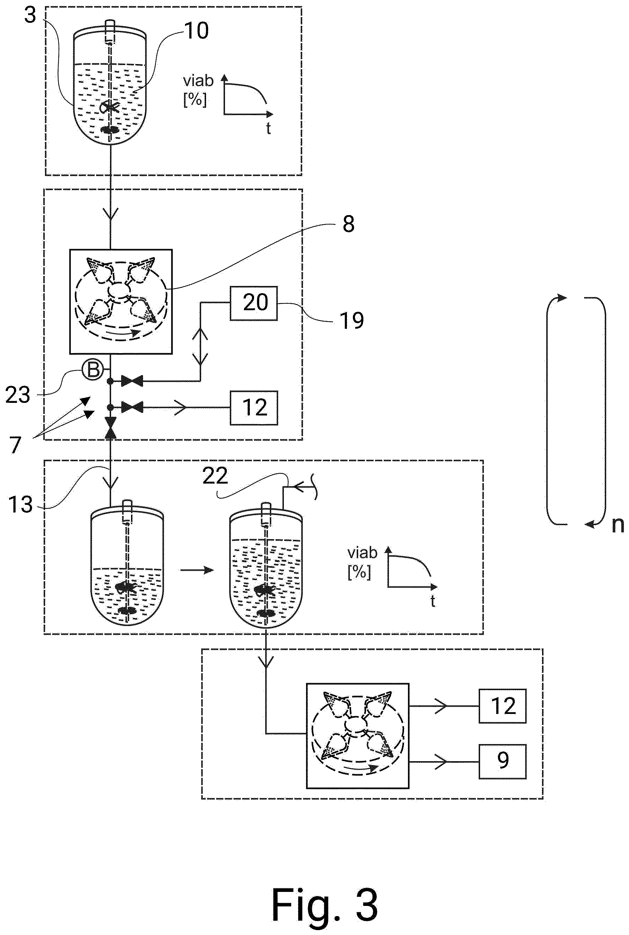

Here and preferably, the bioprocessing arrangement 1 comprises a valve arrangement 7. At least one valve of the valve arrangement 7 may be switched during a transition from the loading step 14 to the overloading step 15. Preferably and as shown in Figs. 1 and 3, said valve is fluidically connected to a chamber outlet 17 of the centrifuge chamber 11 such that switching the valve reroutes a fluid output of the centrifuge chamber 11. The fluid output of the chamber is routed differently during the loading step 14 and the overloading step 15 such that the fluid output during the overloading step 15 is collected as the waste fraction 12 and the fluid output during the loading step 14 is collected separately. Here and preferably, the fluid output during the loading step 14 is collected in a holding vessel 19 shown in Fig. 1. Here and preferably, the valve arrangement 7 is controlled by the control system 6, in particular such that the separation of the cell broth 10 may be performed automatically without user intervention.

The fluid output during the loading step 14 is here and preferably collected as a supernatant fraction 20, preferably in a holding vessel 19 as shown in Fig. 1. Preferably, the waste fraction 12 comprises at least 10, preferably at least 100, more preferably at least 1,000, times more cells per mL than the supernatant fraction 20.

According to one embodiment it is proposed that during the loading and overloading step 15 cell broth 10 is input into the chamber in a forward direction, that the separation of the cell broth 10 into the waste fraction 12 and the output fraction 13 comprises a collection step 21 during which the cells are flushed out from the chamber in a reverse direction opposite to the forward direction and collected as part of the output fraction 13 (Fig. 2 c)).

Here and preferably and as can be seen from the fluid line connections in the figures, during the collection step 21 supernatant from the supernatant fraction 20, in particular from the holding vessel 19, is pumped through the chamber in the reverse direction thereby flushing out the cells from the chamber. In this way, it is possible to operate the fluidized bed centrifuge 8 and perform the separation without needing buffer. The whole supernatant fraction 20 or a part of it may be returned into the vessel, in particular during the reverse operation. Alternatively to the supernatant fraction 20, fresh culture medium 22 may be used thereby directly feeding the cells in the vessel.

During forward operation, cell broth 10 or liquid in general is pumped from the chamber inlet 16 to the chamber outlet 17, during reverse operation liquid is pumped in the opposite direction.

Turning towards the overall process, according to one embodiment and as already explained it is proposed that the cell broth 10 is extracted from a vessel, in particular bioreactor 3, of the bioprocessing arrangement 1 prior to the loading step 14, that the output fraction 13 is returned to the vessel, preferably, that at least 50%, preferably at least 75%, more preferably at least 90%, of the supernatant fraction 20 are returned to the vessel. Alternatively, the product 9 may be harvested from the supernatant fraction 20. In Fig. 1, a separate fluid line for returning the output fraction 13 or the supernatant fraction or part of the supernatant fraction to the bioreactor 3 is shown. In another embodiment, a single fluid line may be used to extract cell broth 10 from the reactor and to return the output fraction 13 to the bioreactor 3. The valve arrangement 7 can be adapted accordingly.

Generally, a batch or fed-batch or continuous process may be performed in the vessel to produce a product 9, the product 9 being produced by the cells or the product 9 being the cells. Here and preferably, the production of the product 9 is continued to be produced in the vessel after returning the output fraction 13 to the vessel.

It may be the case that the separation of cell broth 10 from the vessel into the waste fraction 12 and the output fraction 13 is done repeatedly with fractions of the cell broth 10 from the vessel during an interval, in particular an interval of up to 8 hours, thereby increasing the vitality of the cells in the vessel. Preferably, the separation is repeated at least for another such interval, preferably for at least another two such intervals. After each interval, single use components of the fluidized bed centrifuge 8 may be changed.

For example, an amount of at least 20 %, preferably at least 30 %, and/or up to 100 % of the volume of the bioreactor 3 may be successively separated into the waste fraction 12 and the output fraction 13 by the fluidized bed centrifuge 8 during the interval. This may take for example 1 hour. After the interval of for example 6 hours, the process may be repeated, thereby revitalizing for example a third of the volume every 6 hours. Fig. 3 shows schematically the repetition of the process. In Fig. 3 at the top, the bioreactor 3 is operated until the viability reaches a threshold or until the end of the interval. At least some cell broth 10 is then subjected to the separation into the waste fraction 12 and the output fraction 13 and added back into the bioreactor 3 in the third row of Fig. 3. This process is repeated until harvest at the bottom of Fig. 3.

According to one embodiment it is proposed that fresh culture medium 22 is added to the vessel after more than one repetition of the separation, preferably cyclically in steps of more than one repetition and/or in steps of at least one interval.

Another aspect of the proposed method is that the separation of the cell broth 10 into the waste fraction 12 and the output fraction 13 may comprise a priming step prior to the loading step 14 during which the centrifuge chamber 11 is primed. It is generally known to prime a fluidized bed centrifuge 8 chamber with buffer prior to loading. Here and preferably however, the chamber is primed with cell broth 10. In this embodiment, no buffer is needed for priming. The fluid output of the centrifuge chamber 11 during the priming step may be directly or indirectly returned into the vessel. Figs. 1 and 3 show respective valves which can be actuated accordingly. Here and preferably, during the transition from the priming step to the loading step 14, a valve of the valve arrangement 7 is switched. Fig. 1 also shows that, as no buffer is needed here, it may be the case that two inlets of the fluidized bed centrifuge 8 are connected to the vessel.

According to one embodiment it is proposed that the bioprocessing arrangement 1 comprises a biomass sensor 23, in particular capacitive biomass sensor 23, measuring a cell viability of the cells in the fluid output of the centrifuge chamber 11. Preferably, the overloading step 15 is stopped based on the signal from the biomass sensor 23. If the biomass sensor 23 detects a sharp increase in viability of the cells in the fluid output or detects that the viability reached a threshold, the overloading step 15 may be stopped.

It is further possible to include another biomass sensor 23 in the vessel or between the vessel and the fluidized bed centrifuge 8. The overloading step 15 may then be stopped based on the signals from both biomass sensors 23.

In an alternative embodiment it is proposed that during the overloading step 15 the maximum cell loading capacity of the chamber is exceeded by a predefined amount of cells added to the chamber. Preferably, the predefined amount depends on a viability measurement of the cells of the cell broth 10. More preferably, the predefined amount is between 80 % and 120 %, preferably between 90 % and 110 %, of the number of dead cells expected to be in the centrifuge chamber 11 based on the viability measurement. Independently, the number of separation steps per interval may depend on the viability measurement.

As shown in the figures, the bioprocessing arrangement 1 may comprise a bioreactor control unit 4 for controlling the production in the vessel and/or a centrifuge control unit 5 for controlling the centrifuge and preferably the valve arrangement 7. Both control units may be part of the control system 6 as explained.

The bioreactor control unit 4 and the centrifuge control unit 5 may be part of one control unit or communicatively coupled to control automatically retrieving the cell broth 10 from the vessel, separating the cell broth 10 and returning the output fraction 13 and optionally the supernatant fraction 20 to the vessel repeatedly. The bioreactor control unit 4 and/or the centrifuge control unit 5 and/or the control system 6 may comprise one or more interfaces to control one or more pumps and/or one or more valves of the valve arrangement 7 and comprise at least a processor configured to perform the named operations.

According to one embodiment it is proposed that rotation speed of the centrifuge chamber 11 and flow speed at the input of the centrifuge chamber 11 are not substantially or not at all changed between the loading step 14 and the overloading step 15. Here and preferably, no washing step is performed during the separation of the cell broth 10.

The term "not substantially changed" here means that the fluidized bed is not disturbed in a manner that would destabilize the fluidized bed.

According to one embodiment it is proposed that the vessel has a volume of at least 1,000 L, preferably at least 1,500 L, more preferably at least 2,000 L, and/or, that the cell broth 10 comprises more than 25,000,000 cells per mL, preferably more than 40,000,000 cells per mL, preferably, that the cells are CHO cells.

Another teaching which is of equal importance relates to a control system 6 configured to control a bioprocessing arrangement 1 to perform the proposed method.

All explanations given with regard to the proposed method are fully applicable.

## Claims

1. Method of operating a bioprocessing arrangement (1) comprising a clarification set-up (2) to remove cell debris from a cell broth (10), wherein the clarification set-up (2) comprises a fluidized bed centrifuge (8) with at least one centrifuge chamber (11), wherein the cell broth (10) is separated into at least a waste fraction (12) and an output fraction (13), wherein the separation of the cell broth (10) into the waste fraction (12) and the output fraction (13) comprises
a loading step (14) during which the centrifuge chamber (11) is loaded with cells of the cell broth (10) up to a capacity below or equal to a maximum cell loading capacity of the centrifuge chamber (11),
an overloading step (15) during which the centrifuge chamber (11) is further loaded with cell broth (10), thereby flushing out cells from the centrifuge chamber (11),
collecting cells flushed out during the overloading step (15) as the waste fraction (12), and
collecting cells from the chamber as the output fraction (13).

2. Method according to claim 1, **characterized in that** the bioprocessing arrangement (1) comprises a valve arrangement (7), that at least one valve of the valve arrangement (7) is switched during a transition from the loading step (14) to the overloading step (15), preferably, that said valve is fluidically connected to a chamber outlet (17) of the centrifuge chamber (11) such that switching the valve reroutes a fluid output of the centrifuge chamber (11), that the fluid output of the chamber is routed differently during the loading step (14) and the overloading step (15) such that the fluid output during the overloading step (15) is collected as the waste fraction (12) and the fluid output during the loading step (14) is collected separately.

3. Method according to claim 2, **characterized in that** the fluid output during the loading step (14) is collected as a supernatant fraction (20), preferably in a holding vessel (19), preferably, that the waste fraction (12) comprises at least 10, preferably at least 100, more preferably at least 1,000, times more cells per mL than the supernatant fraction (20).

4. Method according to one of the preceding claims, **characterized in that** during the loading and overloading step (15) cell broth (10) is input into the chamber in a forward direction, that the separation of the cell broth (10) into the waste fraction (12) and the output fraction (13) comprises a collection step (21) during which the cells are flushed out from the chamber in a reverse direction opposite to the forward direction and collected as part of the output fraction (13), preferably, that during the collection step (21) supernatant from the supernatant fraction (20), in particular from the holding vessel (19), is pumped through the chamber in the reverse direction thereby flushing out the cells from the chamber.

5. Method according to one of the preceding claims, **characterized in that** the cell broth (10) is extracted from a vessel, in particular bioreactor (3), of the bioprocessing arrangement (1) prior to the loading step (14), that the output fraction (13) is returned to the vessel, preferably, that at least 50%, preferably at least 75%, more preferably at least 90%, of the supernatant fraction (20) is returned to the vessel, or, that a product (9) is harvested from the supernatant fraction (20).

6. Method according to claim 5, **characterized in that** a batch or fed-batch or continuous process is performed in the vessel to produce a product (9), the product (9) being produced by the cells or the product (9) being the cells, that the production of the product (9) is continued to be produced in the vessel after returning the output fraction (13) to the vessel.

7. Method according to claim 6, **characterized in that** the separation of cell broth (10) from the vessel into the waste fraction (12) and the output fraction (13) is done repeatedly with fractions of the cell broth (10) from the vessel during an interval, in particular an interval of up to 8 hours, thereby increasing the vitality of the cells in the vessel, preferably, that the separation is repeated at least for another such interval, preferably for at least another two such intervals.

8. Method according to claim 7, **characterized in that** fresh culture medium (22) is added to the vessel after more than one repetition of the separation, preferably cyclically in steps of more than one repetition and/or in steps of at least one interval.

9. Method according to one of the preceding claims, **characterized in that** the separation of the cell broth (10) into the waste fraction (12) and the output fraction (13) comprises a priming step prior to the loading step (14) during which the centrifuge chamber (11) is primed, preferably, that the chamber is primed with cell broth (10).

10. Method according to one of the preceding claims, **characterized in that** the bioprocessing arrangement (1) comprises a biomass sensor (23), in particular capacitance biomass sensor (23), measuring a cell viability of the cells in the fluid output of the centrifuge chamber (11), preferably, that the overloading step (15) is stopped based on the signal from the biomass sensor (23).

11. Method according to one of the preceding claims, **characterized in that** during the overloading step (15) the maximum cell loading capacity of the chamber is exceeded by a predefined amount of cells added to the chamber, preferably, that the predefined amount depends on a viability measurement of the cells of the cell broth (10), more preferably, that the predefined amount is between 80 % and 120 %, preferably between 90 % and 110 %, of the number of dead cells expected to be in the centrifuge chamber (11) based on the viability measurement.

12. Method according to one of the preceding claims, **characterized in that** the bioprocessing arrangement (1) comprises a bioreactor control unit (4) for controlling the production in the vessel and/or a centrifuge control unit (5) for controlling the centrifuge and preferably the valve arrangement (7), preferably, that the bioreactor control unit (4) and the centrifuge control unit (5) are part of one control unit or communicatively coupled to control automatically retrieving the cell broth (10) from the vessel, separating the cell broth (10) and returning the output fraction (13) and optionally the supernatant fraction (20) to the vessel repeatedly.

13. Method according to one of the preceding claims, **characterized in that** rotation speed of the centrifuge chamber (11) and flow speed at the input of the centrifuge chamber (11) are not substantially or not at all changed between the loading step (14) and the overloading step (15), and/or, that no washing step is performed during the separation of the cell broth (10).

14. Method according to one of the preceding claims, **characterized in that** the vessel has a volume of at least 1,000 L, preferably at least 1,500 L, more preferably at least 2,000 L, and/or, that the cell broth (10) comprises more than 25,000,000 cells per mL, preferably more than40,000,000 cells per mL, preferably, that the cells are CHO cells.

15. Control system configured to control a bioprocessing arrangement (1) to perform the method according to one of the preceding claims.
